# EUROPÄISCHE PATENTSCHRIFT

(11) **EP 2 055 328 B1**
(45) Veröffentlichungstag und Bekanntmachung des Hinweises auf die Patenterteilung: **12.05.2010**
(21) Anmeldenummer: 08164997.2
(22) Anmeldetag: 24.09.2008
(51) Int. Cl.: A61L 31/08, A61F 2/86, A61F 2/82, A61F 2/00

(54) **Stent mit einem Grundkörper aus einem bioinerten metallischen Implantatwerkstoff**
Stent with a base body made of a bioinert metallic implant material
Stent doté d'un corps de base constitué d'une matière bioinerte métallique

(30) Priorität: 24.10.2007 DE 102007050668
(43) Veröffentlichungstag der Anmeldung: 06.05.2009
(73) Patentinhaber: Biotronik VI Patent AG, 6341 Baar (CH)
(72) Erfinder: Harder, Claus, 91080, Uttenreuth (DE); Borck, Alexander, 91086, Aurachtal (DE); Bayer, Gerd, 91054, Erlangen (DE); Fringes, Matthias, 91522, Ansbach (DE)
(74) Vertreter: Lindner-Vogt, Karin L.

(56) Entgegenhaltungen:
- WO-A-03/086496
- US-A1- 2005 238 689
- US-A1- 2005 271 701
- US-A1- 2007 203 573

## Beschreibung

Die Erfindung betrifft einen Stent mit einem Grundkörper aus einem bioinerten metallischen Implantatwerkstoff.

### Technologischer Hintergrund und Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen rohrförmigen Grundkörper mit einer filigranen Tragstruktur aus metallischen Streben auf, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Der Grundkörper des Stents besteht aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatwerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatwerkstoffe in bioaktive, bioinerte und degradierbare/resorbierbare Werkstoffe unterteilt werden. Für die Zwecke der vorliegenden Erfindung sind lediglich bioinerte, genauer permanente, metallische Implantatwerkstoffe für Stents von Interesse.

Eine biologische Reaktion auf metallische Elemente hängt ab von der Konzentration, Einwirkdauer und Art der Zuführung. Häufig führt allein die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität und damit eine Verbesserung der Restenoserate erreichen lässt, wenn metallische Implantatwerkstoffe mit Beschichtungen aus besonders gewebeverträglichen Materialien versehen werden. Diese Materialien sind zumeist organischer oder synthetisch-polymerer Natur und teils natürlichen Ursprungs.

Eine weitere Strategie zur Vermeidung der Restenose konzentriert sich darauf, die Proliferation durch Medikation zu hemmen. Es sind wirkstoffbeschichtete Stents (auch als DES bezeichnet: drug eluting stent) bekannt, die sehr potent die Proliferation glatter humaner Gefäßmuskelzellen unterbinden; Beispiele umfassen mit den Wirkstoffen Sirolimus oder Paclitaxel beschichtete Stents.

Nachteilig bei bekannten DES ist, dass durch die unspezifische Wirkungsweise der bisher verwendeten, antiproliferativen Substanzen auch die Endothelialisierung behindert beziehungsweise unterbunden wird [M. Joner, A. V. Finn, A. Farb, E. K. Mont, F. D. Kolodgie, E. Ladich, R. Kutys, K. Skorija, H. K. Gold, and R. Virmani. Pathology of drugeluting stents in humans: delayed healing and late thrombotic risk. J.Am.Coll.Cardiol. 48 (1):193-202, 2006]. Eine mangelnde Endothelialisierung führt nach Ansicht der klinischen Experten zu einem höheren Risiko des Auftretens von In-Stent-Thrombosen, die in etwa der Hälfte der Fälle tödlich verlaufen.

Ferner haben bekannte DES den Nachteil, dass das Einwachsverhalten in die Gefäßwand verzögert verläuft [A. V. Finn, G. Nakazawa, M. Joner, F. D. Kolodgie, E. K. Mont, H. K. Gold, and R. Virmani. Vascular responses to drug eluting stents: importance of delayed healing. Arterioscler. Thromb. Vasc. Biol. 27 (7):1500-1510, 2007]. Die antiproliferative Wirkung der heute gängigen DES-Substanzen unterdrückt die Ausbildung einer Neointima nahezu vollständig. Dadurch wird zwar die Restenosierung erfolgreich inhibiert, aber eben auch das Einwachsen des Stents in die Gefäßwand. Dabei lässt sich häufig beobachten, dass der implantierte Stent - wenigstens partiell - nicht mehr in Kontakt mit der Gefäßwand steht. Daraus können sich Strömungsturbulenzen mit Thrombusbildung sowie Aussackungen (Aneurysmen) der Gefäßwand bilden, die im schlimmsten Fall rupturieren können.

Schließlich verwenden die meisten bekannten DES permanente Polymere als Wirkstoff-Reservoirs, die wenig biokompatibel sind und zum Teil für das Auftreten der Spät-Thrombosen mit verantwortlich gemacht werden können.

Infolge dieser drei Nachteile ergibt sich bei herkömmlichen DES insbesondere das Risiko einer gegenüber reinen metallischen Stents erhöhten Rate später In-Stent-Thrombosen, die zu etwa der Hälfte der Fälle tödlich verlaufen. Aus WO 03/086496 geht ein DES hervor, dessen Oberfläche eine hämokompatible Beschichtung aus Siliziumkarbid und einen antiproliferativen Wirkstoff aufweist. Diese Oberflächenbehandlung eignet sich sowohl für die Außenflächen wie auch für Hohlräume des Implantats, wenn solche Vorhanden sind.

Trotz der erreichten Fortschritte ist daher eine weitere Verbesserung der Integration des Stents in sein biologisches Umfeld und dadurch Senkung der Restenoserate wünschenswert.

### Erfindungsgemäße Lösung

Die Erfindung geht aus von einem Stent mit einem Grundkörper aus einem bioinerten metallischen Implantatwerkstoff. Der erfindungsgemäße Stent zeichnet sich dadurch aus, dass
(i) der Grundkörper mit einer SiC-Beschichtung bedeckt ist;
(ii) eine murale Oberfläche des Grundkörpers eine Vielzahl von Kavitäten aufweist, die mit einem antiproliferativen Wirkstoff befüllt sind; und
(iii) eine luminale Oberfläche des Grundkörpers eine Beschichtung aufweist, die Attraktoren für Endothelzellen enthält oder aus diesen besteht.

Der Erfindung liegt die Erkenntnis zugrunde, dass eine Kombination aus einer SiC-Beschichtung des Grundkörpers, eines in eine Vielzahl von Kavitäten eingebrachten antiproliferativen Wirkstoffs auf der Außenseite des Stents und einer Beschichtung mit einer Attraktor für Endothelzellen auf der luminalen Seite des Grundkörpers zu einem Stent mit für den Verwendungszweck optimalen Eigenschaften führt. Bisherige Ansätze der Stentoptimierung mit wirkstoffbeschichteten Stents richten sich vornehmlich auf die Auswahl der Wirksubstanz. Andere Faktoren, wie das geometrische Design und mechanische Parameter der Grundstruktur, spielen praktisch keine Rolle.

Als Implantatwerkstoff für den Grundkörper werden vorzugsweise CoCr, 316L oder Nitinol eingesetzt. Der Stent kann in Form einer filigranen Tragstruktur vorliegen, vorzugsweise mit einem Helixdesign, wie beispielsweise aus EP 1 430 854 A bekannt.

Der Grundkörper des Stents ist mit einer halbleitenden, isolierenden und passivierenden SiC-Schicht bedeckt. In Langzeitversuchen hat es sich gezeigt, dass SiC-beschichtete Implantate mit Endothelzellen bewachsen werden und eine weitaus hydrophilere Oberfläche besitzen als herkömmliche Stents. Hierdurch kann auch die Haftfestigkeit der an luminaler und muraler Seite ausgebrachten Wirkstoffe/Attraktoren erhöht werden.

Die murale Oberfläche (Außenoberfläche) des Stents weist eine Vielzahl von Kavitäten auf. Diese Vertiefungen der Außenoberfläche des Stents haben Abmessungen von wenigen Mikrometern und lassen sich beispielsweise über Laserverfahren herstellen. Die Einbringung von Wirkstoffen in Kavitäten hat den Vorteil, dass auf eine Beschichtung, die als Matrix zum Einbetten der Wirkstoffe dient, verzichtet werden kann. Häufig sind die Bestandteile derartiger Matrices oder deren Abbauprodukte Ursache von Unverträglichkeitsreaktionen, die Ausgangspunkt restenotischer Prozesse sein können. Dies trifft insbesondere für die meisten in der Praxis eingesetzten polymeren Matrices zu. Die genaue Anzahl, Lage, Kontur und Ausführung der Kavitäten wird der Fachmann anhand des ihm zur Verfügung stehenden Stentdesigns und der Menge des einzubringenden Wirkstoffs in einfacher Weise festlegen können. Bevorzugt sind Sacklöcher entsprechend der Ausführung in WO 2006/133223 A.

Die in die Kavitäten eingebrachte antiproliferative Substanz wird nach der Implantation ausschließlich in die Gefäßwand eluiert und von der luminalen Oberfläche weitgehend ferngehalten. Dadurch behindert die Substanz die Endothelialisierung praktisch nicht. Vorzugsweise ist der antiproliferative Wirkstoff ausgewählt aus den Taxolen und Taxanen, vorzugsweise aus der Gruppe Paclitaxel, Sirolimus sowie insbesondere Derivaten des Rapamycin, wie Everolimus oder Biolomus. Sirolimus und die Derivate des Rapamycin weisen eine im Vergleich zu anderen auf Stents aufgebrachten Wirkstoffen verzögerte Freisetzungskinetik auf und sorgen somit dafür, dass der Wirkstoff, auch ohne eine der Verzögerung der Freisetzung dienende Matrix/Überzug Einsatz finden kann.

Weiterhin fördert die SiC-Schicht auf der luminalen Seite die Anhaftung des Wirkstoffes und im Nachgang das Einwachsen in die Gefäßwand und unterbindet negative Effekte durch ihre hohe Biokompatibilität.

Die luminale Oberfläche des Stents ist ebenfalls mit der SiC-Schicht bedeckt, was nachgewiesener Maßen eine rasche Endothelialisierung fördert. Vorzugsweise ist die luminale Oberfläche des Grundkörpers zur Oberflächenvergrößerung konturiert, dass heißt, weist eine Textur auf. Vorzugsweise sollte die Textur der luminalen Oberfläche so gestaltet sein, dass die Anlagerung von Endothelzellen gefördert wird. Diese Texturen können durch Aufrauen der Oberfläche erzeugt werden. Dies geschieht z. B. durch Sputtern, Lasern oder Passivieren. Besonders bevorzugt ist eine durch Sputtern hergestellte Textur, die im gleichen Prozess mit der SiC-Schicht beschichtet wird.

Schließlich befinden sich auf luminaler Seite eine Beschichtung, die Attraktoren mit endothelspezifischen Bindungsmöglichkeiten enthält oder aus diesen besteht. Als Attraktoren werden in diesem Zusammenhang in kovalenter Weise an die Oberfläche angebundene chemische Einheiten betrachtet, die die bevorzugte Anbindung von Endothelzellen ermöglichen. Die Attraktoren umfassen insbesondere Antikörper (wie z. B. CD133), Aptamere, oder RGD-Peptide, insbesondere zyklische RGD's (cRGD).

Auf diese Weise wird eine rasche und sichere Endothelialisierung erreicht, die durch ihre physiologische Funktionalität, zum Beispiel NO-Emission, zudem antiproliferativ wirkt. Derzeitige - zum Beispiel mit Sirolimus oder Paclitaxel - beschichtete Stents verhindern oder zumindest erschweren eine Endothelialisierung auf luminaler Seite. Daher ist eine begleitende medikamentöse Therapie zur Thromboseprophylaxe über viele Jahre notwendig. Dieser Nachteil kann mit Hilfe des erfindungsgemäßen Stents überwunden werden. Nach der vollständigen Abgabe des Medikaments und der Endothelialisierung bleibt im Gefäß lediglich ein mit SiC-beschichteter permanenter Stent zurück. SiC hat sich über mehr als zehn Jahr als hochgradig biokompatibel erwiesen.

Durch das Einbringen der Wirkstoffe in Kavitäten auf der muralen Seite kann eine weitgehende polymerfreie Stentbeschichtung realisiert werden. Der Wirkstoff kann in bekannter Weise gezielt in die Kavitäten eingebracht werden. Denkbar ist zum Beispiel zur zeitlichen Verzögerung der Wirkstofffreisetzung, die Wirkstoffe entsprechend üblicher Galenik zu verkapseln. Die Galenik ist ein Teilgebiet der pharmazeutischen Technologie und beschäftigt sich mit der Darreichungsform der Medikamente. Klassisch bestand eine Aufgabe darin empfindliche Wirkstoffe vor der Magensäure zu schützen. Das wurde realisiert, indem säurestabile aber basisch anfällige Polymere wie Eurdagit benutzt wurden, um Tabletten zu umhüllen. Es hat sich herausgestellt, dass dieser Grundgedanke auch für Stents interessant ist, da Wirkstoffe wie Rapamycin über längere Zeiträume abgegeben werden sollen. Insbesondere ist die Galenik hilfreich, wenn, wie in der vorliegenden Anmeldung beschrieben, die mit Wirkstoff gefüllten Kavitäten galenisch "verkapselt" werden (z. B. mit Eurdagit).

Nachfolgend einige bevorzugte Ausführungsbeispiele:
1) Rapamycin wird mit Chloroform in Lösung gebracht und mit beta-Cyclodextrin in Wasser ausgeschüttelt.
2) Rapamycin wird zusammen mit einem Polylactid (zum Beispiel unter den Handelsnamen RESOMER L203 H oder RESOMER L 203 S erhältliche Polylactide) in Chloroform gelöst und sprühgetrocknet.
3) Herstellung eines Gemisches aus Rapamycin mit 6-O-Palmitoyl-L-ascorbinsäure oder Polyvinylpyrolidon. Die Mischung kann als Lösung oder als Pressling in die Kavitäten gebracht werden.

In einer weiteren bevorzugten Ausführungsform werden die Kavitäten mit einer oder mehreren der Substanzen aus der Gruppe Zucker, wie Polysaccharide, Glykane, Glucose, Glycogen, Amylose, Amylopektin, Chitin, Callose und Zellulose und Fette, wie Cholesterin, Cholesterol, Palmöl, partiell hydrierte Sojaöle und gesättigte Öle überzogen/besch ichtet.

Die Erfindung wird nachfolgend anhand eines Ausführungsbeispiels und der dazugehörigen Zeichnung erläutert.

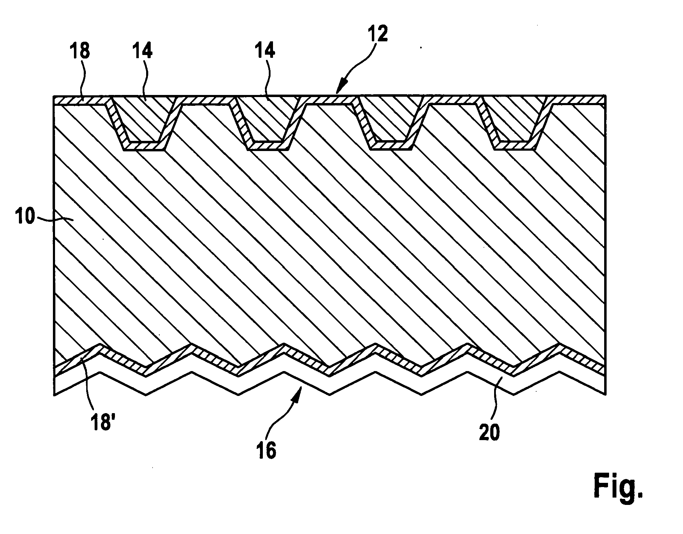

Die einzige Zeichnung zeigt - stark schematisiert - einen Schnitt durch eine Strebe der Tragstruktur des Stents. Ein Grundkörper 10 aus einem permanenten metallischen Implantatwerkstoff (beispielsweise CoCr) weist auf seiner muralen Oberfläche 12 eine Vielzahl von Kavitäten 14 auf, die sich mit gängigen Bearbeitungsverfahren erzeugen lassen (zum Beispiel mittels eines Laserstrahls). Der dargestellte Schnitt läuft durch einige der Kavitäten 14 auf der muralen Oberfläche 12. Die Abmessungen der Kavitäten 14 liegen im Bereich von wenigen Mikrometern.

Auch eine luminale Oberfläche 16 des Grundkörpers 10 ist texturiert. Sie weist - wie hier beispielhaft dargestellt - eine wellenförmige Kontur auf, die zu einer Oberflächenvergrößerung der luminalen Oberfläche 16 führt und dementsprechend sich über weite Teile oder die gesamte luminale Oberfläche 16 erstreckt.

Sowohl die murale Oberfläche 12 als auch die luminale Oberfläche 16 sind von einer SiC-Schicht 18, 18' bedeckt. Die Herstellung solcher SiC-Schichten 18, 18' ist aus dem Stand der Technik bekannt, sodass auf eine ausführliche Beschreibung an dieser Stelle verzichtet wird.

Die Kavitäten 14 sind mit einer antiproliferativen Substanz befüllt (z. B. Paclitaxel). Auf luminaler Seite ist eine Beschichtung 20 mit Attraktoren für Endothelzellen vorhanden.

## Patentansprüche

1. Stent mit einem Grundkörper (10) aus einem bioinerten metallischen Implantatwerkstoff, **dadurch gekennzeichnet, dass**
(i) der Grundkörper (10) mit einer SiC-Beschichtung (18, 18') bedeckt ist;
(ii) eine murale Oberfläche (12) des Grundkörpers (10) eine Vielzahl von Kavitäten (14) aufweist, die mit einem antiproliferativen Wirkstoff befüllt sind; und
(iii) eine luminale Oberfläche (16) des Grundkörpers (10) eine Beschichtung (20) aufweist, die Attraktoren für Endothelzellen enthält oder aus diesen besteht.

2. Stent nach Anspruch 1, bei dem die luminale Oberfläche (16) des Grundkörpers (10) konturiert ist.

3. Stent nach einem der Ansprüche 1 oder 2, bei dem der Grundkörper (10) aus CoCr, 316L oder Nitinol besteht.

4. Stent nach einem der vorhergehenden Ansprüche, bei dem der antiproliferative Wirkstoff ausgewählt ist aus den Taxolen und Taxanen, vorzugsweise aus der Gruppe Paclitaxel, Sirolimus , sowie insbesondere Derivaten des Rapamycin, wie Everolimus oder Biolomus.

5. Stent nach einem der vorhergehenden Ansprüche, bei dem der Attraktor für Endothelzellen ausgewählt ist aus der Gruppe Antikörper, wie z. B. CD133, Aptamere, oder RGD-Peptide, insbesondere zyklische RGD's, das heißt cRGD.

## Claims

1. A stent comprising a main body (10) made of a bioinert metal implant material,
**characterized in that**
(i) the main body (10) is covered with an SiC coating (18, 18');
(ii) a mural surface (12) of the main body (10) has a plurality of cavities (14) which are filled with an antiproliferative active ingredient; and
(iii) a luminal surface (16) of the main body (10) has a coating (20) which comprises or is made of attractors for endothelial cells.

2. The stent according to claim 1, wherein the luminal surface (16) of the main body (10) is contoured.

3. The stent according to claim 1 or 2, wherein the main body (10) comprises CoCr, 316L, or nitinol.

4. A stent according to any one of the preceding claims, wherein the antiproliferative active ingredient is preferably selected from taxols and taxanes, preferably from the group consisting of paclitaxel, sirolimus and in particular the derivatives of rapamycin, such as everolimus or biolomus.

5. A stent according to any one of the preceding claims, wherein the attractor for endothelial cells is selected from the group consisting of antibodies, such as CD133, aptamers, or RGD peptides, in particular cyclic RGDs, which is to say cRGD.

## Revendications

1. Stent, avec un corps de base (10) fait dans un matériau métallique bioinerte pour implant, **caractérisé par le fait que**:
(i) le corps de base (10) est recouvert d'un revêtement de SiC (18, 18');
(ii) une surface murale (12) du corps de base (10) présente une pluralité de cavités (14) qui sont remplies d'un agent antiprolifératif; et
(iii) une surface luminale (16) du corps de base (10) présente un revêtement (20) contenant des attracteurs pour cellules endothéliales ou constitué de ceux-ci.

2. Stent selon la revendication 1, dans lequel la surface luminale (16) du corps de base (10) possède un contour.

3. Stent selon l'une des revendications 1 ou 2, dans lequel le corps de base (10) est fait de CoCr, de 316L ou de nitinol.

4. Stent selon l'une des revendications précédentes, dans lequel l'agent antiprolifératif est choisi parmi les taxols et les taxanes, de préférence dans le groupe composé du paclitaxel, du sirolimus, ainsi qu'en particulier, de dérivés de la rapamycine tels que l'everolimus ou le biolomus.

5. Stent selon l'une des revendications précédentes, dans lequel l'attracteur de cellules endothéliales est choisi dans le groupe des anticorps, tels que, par exemple, le CD133, des aptamères ou des peptides RGD, en particulier des RGD cycliques, notés RGDc.
